# EUROPEAN PATENT APPLICATION

(11) **EP 2 636 409 A1**
(43) Date of publication of application: **11.09.2013**
(21) Application number: 11838098.9
(22) Date of filing: 04.11.2011
(51) Int. Cl.: A61K 31/444, A61K 31/4174, A61K 31/4196, A61K 31/427, A61K 31/496, A61K 31/505, A61K 31/506, A61K 31/7048, A61K 38/00, A61K 45/00, A61P 31/10, A61P 43/00

(54) **COMBINED PHARMACEUTICAL COMPOSITION AS ANTIFUNGAL AGENT**

(30) Priority: 25.08.2011 JP 2011183874; 05.11.2010 JP 2010248337
(71) Applicant: Eisai R&D Management Co., Ltd., Tokyo 112-8088 (JP)
(72) Inventor: WATANABE, Naoaki, Tsukuba-shi Ibaraki 300-2635 (JP); HATA, Katsura, Tsukuba-shi Ibaraki 300-2635 (JP); MIYAZAKI, Mamiko, Tsukuba-shi Ibaraki 300-2635 (JP); HORII, Takaaki, Tsukuba-shi Ibaraki 300-2635 (JP)
(74) Representative: HOFFMANN EITLE
(86) International application number: PCT/JP2011/075481
(87) International publication number: WO 2012/060448

(57) **Abstract**

An object of the present invention is to provide a pharmaceutical composition having superior antifungal activity. The present invention provides a pharmaceutical composition comprising 3-(3-(4-(pyridin-2-yloxymethyl)-benzyl)-isoxazol-5-yl)-pyridin-2-ylamine or 2-amino-1-((phosphonooxy)methyl)-3-(3((4-((2-pyridinyloxy)methyl)phenyl)methyl)-5-isoxazolyl)-pyridinium or a salt thereof in combination with an antifungal agent.

## Description

### Technical Field

The present invention relates to a combined pharmaceutical composition as an antifungal agent. More particularly, the present invention relates to a pharmaceutical composition and kit comprising 3-(3-(4-(pyridin-2-yloxymethyl)-benzyl)-isoxazol-5-yl)-pyridin-2-ylamine, 2-amino-1-((phosphonooxy)methyl)-3-(3-((4-((2-pyridinyloxy)methyl)phenyl)methyl)-5-isoxazolyl)-pyridinium or a salt thereof in combination with an antifungal agent.

### Background Art

In recent years, there has been a growing need for the implementation of measures against opportunistic infections due to the growing number of patients with suppressed immune functions attributable to extensive chemotherapy and the like as well as the elderly. As is indicated by the fact that opportunistic infections caused by different low virulent microorganisms can occur in succession, there is no end to the problem of infections in such patients as long as there are underlying diseases that lower resistance to such infections. Thus, in consideration of the elderly population that will undoubtedly grow in the future, the development of new infection countermeasures, including those against the problem of drug-resistant microorganisms, is expected to become an important issue.

In the field of antifungal agents, polyene antifungal agents, azole antifungal agents and echinocandin antifungal agents and the like have been developed for the treatment of deep mycosis. In addition, Non-Patent Document 1 discloses that methods using a combination of antifungal agents have been attempted in order to enhance the therapeutic efficacy of these antifungal agents. Moreover, Patent Documents 1 to 3 disclose that research has proceeded on the combination of antifungal agents in recent years.

Under such circumstances, there is an earnest desire for the development of drugs having higher antifungal activity as well as methods for enhancing therapeutic efficacy by combining antifungal agents.

### Prior Art Document

### Patent Document

Patent Document 1: WO 98/10782
Patent Document 2: WO 2008/044562
Patent Document 3: WO 2009/144473

Non-Patent Document 1: Shinzaisei Shinkinsho No Shindan Chiryo Gaidorain (Guideline for the Diagnosis and Treatment of Deep Mycosis), 2003, Ishiyaku Publishers, Inc.

### Summary of the Invention

### Problem to be solved by the Invention

An object of the present invention is to provide a pharmaceutical composition having superior antifungal activity.

### Means for Solving the Problem

As a result of conducting extensive studies in consideration of the above-mentioned circumstances, the inventors of the present invention found that superior antifungal action is demonstrated by using a pharmaceutical composition that combines 3-(3-(4-(pyridin-2-yloxymethyl)-benzyl)-isoxazol-5-yl)-pyridin-2-ylamine or 2-amino-1-((phosphonooxy)methyl)-3-(3-((4-((2-pyridinyloxy)methyl)phenyl)mathyl)-5-isoxazolyl)-pyridinium, or a salt thereof with at least one compound selected from the group consisting of a triazole antifungal agent, an imidazole antifungal agent, an echinocandin antifungal agent, a polyene antifungal agent, a fluoropyridine antifungal agent, T-2307 and FG3622, or a salt thereof, thereby leading to completion of the present invention.

Namely, the present invention is as described below.
[1] A pharmaceutical composition comprising 3-(3-(4-(pyridin-2-yloxymethyl)-benzyl)-isoxazol-5-yl)-pyridin-2-ylamine or 2-amino-1-((phosphonooxy)methyl)-3-(3-((4-((2-pyridinyloxy)methyl)phenyl)methyl)-5-isoxazolyl)-pyridinium, or a salt thereof in combination with an antifungal agent, wherein the antifungal agent is at least one compound selected from the group consisting of a triazole antifungal agent, an imidazole antifungal agent, an echinocandin antifungal agent, a polyene antifungal agent, a fluoropyridine antifungal agent, T-2307 and FG3622, or a salt thereof.
[2] The pharmaceutical composition described in [1], wherein the antifungal agent is at least one compound selected from a triazole antifungal agent, an echinocandin antifungal agent, a polyene antifungal agent and a fluoropyridine antifungal agent, or a salt thereof.
[3] The pharmaceutical composition described in [1] or [2], wherein the antifungal agent is at least one compound selected from a triazole antifungal agent, an echinocandin antifungal agent and a polyene antifungal agent, or a salt thereof.
[4] The pharmaceutical composition described in [1], wherein the antifungal agent is at least one compound selected from the group consisting of fluconazole, fosfluconazole, itraconazole, voriconazole, posaconazole, isavuconazole, ravuconazole, ((2R,3R)-3-(4-(4-cyanophenyl)thiazol-2-yl)-2-(2,4-difluorophenyl)-1-(1H-1,2,4-triazol-1-yl)butan-2-yloxy)methyl dihydrogen phosphate, albaconazole, miconazole, ketoconazole, caspofungin, micafungin, anidulafungin, aminocandin, amphotericin B, nystatin and flucytosine, or a salt thereof.
[5] The pharmaceutical composition described in any one of [1] to [4], wherein the antifungal agent is at least one compound selected from the group consisting of fluconazole, voriconazole, ravuconazole, caspofungin, micafungin and amphotericin B, or a salt thereof.
[6] The pharmaceutical composition described in any one of [1] to [3], wherein the triazole antifungal agent is at least one compound selected from the group consisting of fluconazole, fosfluconazole, itraconazole, voriconazole, posaconazole, isavuconazole, ravuconazole, ((2R,3R)-3-(4-(4-cyanophenyl)thiazol-2-yl)-2-(2,4-difluorophenyl)-1-(1 H-1,2,4-triazol-1-yl)butan-2-yloxy)methyl dihydrogen phosphate and albaconazole, or a salt thereof.
[7] The pharmaceutical composition described in [1], wherein the imidazole antifungal agent is at least one compound selected from the group consisting of miconazole and ketoconazole, or a salt thereof.
[8] The pharmaceutical composition described in any one of [1] to [3], wherein the echinocandin antifungal agent is at least one compound selected from the group consisting of caspofungin, micafungin, anidulafungin and aminocandin, or a salt thereof.
[9] The pharmaceutical composition described in any one of [1] to [3], wherein the polyene antifungal agent is at least one compound selected from the group consisting of amphotericin B and nystatin, or a salt thereof.
[10] The pharmaceutical composition described in [1], wherein the fluoropyridine antifungal agent is flucytosine.
[11] The pharmaceutical composition described in any ane of [1] tao [10], which is used for prevention and/or treatment of mycosis.
[12] The pharmaceutical composition described in any one of [1] to [11], wherein a fungus is Candida or Aspergillus.
[13] The pharmaceutical composition described in [12], wherein the Candida is Candida albicans, Candida glabrata, Candida parapsilosis, Candida tropicalis or Candida krusei.
[14] The pharmaceutical composition described in [12], wherein the Aspergillus is Aspergillus fumigatus, Aspergillus flavus, Aspergillus terreus, Aspergillus niger or Aspergillus nidulans.
[15] The pharmaceutical composition described in any one of [11] to [14], wherein the mycosis is candidiasis or aspergillosis.
[16] The pharmaceutical composition described in any one of [11] to [15], wherein the mycosis is systemic candidiasis, oral candidiasis, esophageal candidiasis, pulmonary candidiasis, urinary candidiasis, pulmonary aspergillosis or central nervous system aspergillosis.
[17] The pharmaceutical composition described in any one of [1] to [16], which is a kit.
[18] A kit comprising 3-(3-(4-(pyridin-2-yloxymethyl)-benzyl)-isoxazol-5-yl)-pyridin-2-ylamine or 2-amino-1-((phasphonooxy)methyl)-3-(3-((4-((2-pyridinyloxy)methyl)phenyl)methyl)-5-isoxazolyl)-pyridinium or a salt thereof in combination with an antifungal agent, wherein the antifungal agent is at least one compound selected from the group consisting of a triazole antifungal agent, an imidazole antifungal agent, an echinocandin antifungal agent, a polyene antifungal agent, a fluoropyridine antifungal agent, T-2307 and FG3622, or a salt thereof.
[19] The kit described in [18], which is for use for prevention and/or treatment of mycosis.
[20] The kit described in [18] or [19], wherein 3-(3-(4-(pyridin-2-yloxymethyl)-benzyl)-isoxazol-5-yl)-pyridin-2-ylamine or 2-amino-1-((phasphonaoxy)methyl)-3-(3-((4-((2-pyridinyloxy)methyl)phenyl)methyl)-5-isoxazolyl)-pyridinium, or a salt thereof, and the antifungal agent are administered simultaneously.
[21] The kit described in [18] or [19], wherein 3-(3-(4-(pyridin-2-yloxymethyl)-benzyl)-isoxazol-5-yl)-pyridin-2-ylamine or 2-amino-1-((phosphonooxy)methyl)-3-(3-((4-((2-pyridinyloxy)methyl)phenyl)methyl)-5-isoxazolyl)-pyridinium, or a salt thereof, and the antifungal agent are administered separately.
[22] A method for prevention and/or treatment of mycosis, comprising administering a pharmacologically effective amount of the pharmaceutical composition described in any one of [1] to [17].
[23] A method for prevention and/or treatment of mycosis, comprising administering a pharmacologically effective amount of 3-(3-(4-(pyridin-2-yloxymethyl)-benzyl)-isoxazol-5-yl)-pyridin-2-ylamine or 2-amino-1-((phosphonooxy)methyl)-3-(3-((4-((2-pyridinyloxy)methyl)phenyl)methyl)-5-isoxazolyl)-pyridinium, or a salt thereof, and a pharmacologically effective amount of an antifungal agent, wherein the antifungal agent is at least one compound selected from the group consisting of a triazole antifungal agent, an imidazole antifungal agent, an echinocandin antifungal agent, a polyene antifungal agent, a fluoropyridine antifungal agent, T-2307 and FG3622, or a salt thereof.
[24] The method described in [23], wherein the pharmacologically effective amount of 3-(3-(4-(pyridin-2-yloxymethyl)-benzyl)-isoxazol-5-yl)-pyridin-2-ylamine or 2-amino-1-((phosphonooxy)methyl)-3-(3-((4-((2-pyridinyloxy)methyl)phenyl)methyl)-5-isoxazolyl)-pyridinium, or a salt thereof, and the pharmacologically effective amount of the antifungal agent are administered simultaneously or separately.
[25] A use of 3-(3-(4-(pyridin-2-yloxymethyl)-benzyl)-isoxazol-5-yl)-pyridin-2-ylamine or 2-amino-1-((phosphonooxy)methyl)-3-(3-((4-((2-pyridinyloxy)methyl)phenyl)methyl)-5-isoxazolyl)-pyridinium, or a salt thereof, and an antifungal agent for the prepetition of a pharmaceutical composition, wherein the antifungal agent is at least one compound selected from the group consisting of a triazole antifungal agent, an imidazole antifungal agent, an echinocandin antifungal agent, a polyene antifungal agent, a fluoropyridine antifungal agent, T-2307 and FG3622, or a salt thereof.
[26] A use of 3-(3-(4-(pyridin-2-yloxymethyl)-benzyl)-isoxazol-5-yl)-pyridin-2-ylamine or 2-amino-1-((phosphonooxy)methyl)-3-(3-((4-((2-pyridinyloxy)methyl)phonyl)methyl)-5-isoxazolyl)-pyridinium, or a salt thereof for the preparation of a pharmaceutical composition in combination with an antifungal agent, wherein the antifungal agent is at least one compound selected from the group consisting of a triazole antifungal agent, an imidazole antifungal agent, an echinocandin antifungal agent, a polyene antifungal agent, a fluoropyridine antifungal agent, T-2307 and FG3622, or a salt thereof.
[27] A combination of 3-(3-(4-(pyridin-2-yloxymethyl)-benzyl)-isoxazol-5-yl)-pyridin-2-ylamine or 2-amino-1-((phosphonooxy)methyl)-3-(3-((4-((2-pyridinyloxy)methyl)phenyl)methyl)-5-isoxazolyl)-pyridinium or a salt thereof, and at least one compound selected from the group consisting of a triazole antifungal agent, an imidazole antifungal agent, an echinocandin antifungal agent, a polyene antifungal agent, a fluoropyridine antifungal agent, T-2307 and FG3622, or a salt thereof.
[28] A combined medicament comprising 3-(3-(4-(pyridin-2-yloxymethyl)-benzyl)-isoxazol-5-yl)-pyridin-2-ylamine or 2-amino-1-((phosphonooxy)methyl)-3-(3-((4-((2-pyridinyloxy)methyl)phenyl)mothyl)-5-isoxazolyl)-pyridinium, or a salt thereof, in combination with an antifungal agent, wherein the antifungal agent is at least one compound selected from the group consisting of a triazole antifungal agent, an imidazole antifungal agent, an echinocandin antifungal agent, a polyene antifungal agent, a fluoropyridine antifungal agent, T-2307 and FG3622, or a salt thereof.
[29] An antifungal agent comprising 3-(3-(4.-(pyridin-2-yloxymethyl)-benzyl)-isoxazol-5-yl)-pyridin-2-ylamine or 2-amino-1-((phosphonooxy)methyl)-3-(3-((4-((2-pyridinyloxy)methyl)phenyl)methyl)-5-isoxazolyl)-pyridinium, or a salt thereof, in combination with at least one compound selected from the group consisting of a triazole antifungal agent, an imidazole antifungal agent, an echinocandin antifungal agent, a polyene antifungal agent, a fluoropyridine antifungal agent, T-2307 and FG3622, or a salt thereof.
[30] A pharmaceutical composition or a kit for use for the method for prevention and/or treatment of mycosis described in [23] or [24].
[31] A medicament comprising 3-(3-(4-(pyridin-2-yloxymethyl)-benzyl)-isoxazol-5-yl)-pyridin-2-ylamine or 2-amino-1-((phosphonooxy)methyl)-3-(3-((4-((2-pyridinyloxy)methyl)phenyl) methyl)-5-isoxazolyl)-pyridinium, or a salt thereof for combination use with at least one compound selected from the group consisting of a triazole antifungal agent, an imidazole antifungal agent, an echinocandin antifungal agent, a polyene antifungal agent, a fluoropyridine antifungal agent, T-2307 and FG3622, or a salt thereof.
[32] A pharmaceutical comprising at least one compound selected from the group consisting of a triazole antifungal agent, an imidazole antifungal agent, an echinocandin antifungal agent, a polyene antifungal agent, a fluoropyridine antifungal agent, T-2307 and FG3622, or a salt thereof for combination use with 3-(3-(4-(pyridin-2-yloxymethyl)-benzyl)-isoxazol-5-yl)-pyridin-2-ylamine or 2-amino-1-((phosphonooxy)methyl)-3-(3-((4-((2-pyridinyloxy)methyl)phenyl)methyl)-5-isoxazolyl)-pyridinium, or a salt thereof.

### Effect of the Invention

The pharmaceutical composition of the present invention has superior antifungal activity and is extremely useful as a preventive or therapeutic agent for mycosis.

### Mode for Carrying Out the Invention

The following provides a detailed explanation of the present invention by indicating the definitions of symbols, terms and the like described in the present description, embodiments of the present invention and the like. Furthermore, the present invention is not limited to the following embodiments, but rather can be carried out while modifying in various ways within the scope of the gist thereof.
In addition, documents cited in the present description are incorporated in the present description by reference to the contents thereof.

In the present description, although structural formulas of compounds only represent a specific isomer for the sake of convenience, the present invention comprises isomers such as all geometrical isomers capable of being formed on the structure of a compound, optical isomers on an asymmetric carbon, stereoisomers, rotational isomers or tautomers and mixtures thereof, is not limited to descriptions of structural formulas indicated for the sake of convenience, and may be any isomer or a mixture thereof. Thus, although a compound of the present invention can have an asymmetric carbon within a molecular thereof and optical isomers and racemic bodies can exist, these are not limited in the present invention and all such forms are comprised therein. In addition, although crystal polymorphism may also exist, this is also similarly not limited in the present invention, and may be a single crystal form or a mixture of two or more crystal forms. Solvates such as anhydrides and hydrates are also comprised in a compound of the present invention.

There are no particular limitations on the term "salt" used in the present description, and a salt may be pharmaceutically acceptable salt or a physiologically acceptable salt.
Although there are no particular limitations on a salt with acid, examples therewith include salts with inorganic acid (such as salts with hydrochloric acid, hydrobromic acid, sulfuric acid, nitric acid or phosphoric acid) and salts with organic acid (such as salts with formic acid, acetic acid, lactic acid, succinic acid, fumaric acid, malic acid, maleic acid, citric acid, tartaric acid, stearic acid, benzoic acid, methanesulfonic acid, ethanesulfonic acid, benzenesulfonic acid, p-toluenesulfonic acid, trifluoroacetic acid, aspartic acid or glutamic acid).
In addition, although there are no particular limitations on a salt with inorganic acid, examples therewith include salts with inorganic base (such as lithium salts, sodium salts, potassium salts, calcium salts or magnesium salts) and salts with organic base (such as salts with methylamine, ethylamine, t-butylamine, trimethylamine, triethylamine, pyridine, picoline, piperidine, morpholine, cyclohexylamine, dicyclohexylamine, N,N'-dibenzylethylenediamine, N-methyl-D-glucamine, arginine, lysine or ornithine).

Although polymorphism may also exist for the term "salt" used in the present description, this is not limited thereto, and may be a single crystal form or a mixture of two or more crystal forms. Solvates such as anhydrides and hydrates are also encompassed in the term "salt" used in the present description.

The pharmaceutical composition of the present invention is a pharmaceutical composition comprising the combination of 3-(3-(4-(pyridin-2-yloxymethyl)-benzyl)-isoxazol-5-yl)-pyridin-2-ylamine or 2-amino-1-((phosphonooxy)methyl)-3-(3-((4-((2-pyridinyloxy)methyl)phenyl)methyl)-5-isoxazolyl)-pyridinium, or a salt thereof in combination with an antifungal agent, wherein the antifungal agent is at least one compound selected from the group consisting of a triazole antifungal agent, an imidazole antifungal agent, an echinocandin antifungal agent, a polyene antifungal agent, a fluoropyridine antifungal agent, T-2307 and FG3622, or a salt thereof.

3-(3-(4-(pyridin-2-yloxymethyl)-benzyl)-isoxazol-5-yl)-pyridin-2-ylamine is a compound described in WO 2007/052615 that is represented by the structure indicated below.

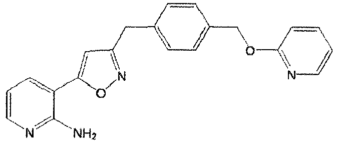

2-amino-1-((phosphonooxy)methyl)-3-(3-((4-((2-pyridinyloxy)methyl)phenyl)methyl)-5-isoxazolyl)-pyridinium is a compound described in WO 2009/084621 that is represented by the structure indicated below.

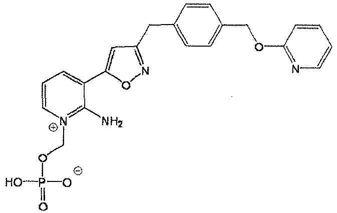

3-(3-(4-(pyridin-2-yloxymethyl)-benzyl)-isoxazol-5-yl)-pyridin-2-ylamine or 2-amino-1-((phosphonooxy)methyl)-3-(3-((4-((2-pyridinyloxy)methyl)phenyl)methyl)-5-isoxazolyl)-pyridinium, or a salt thereof inhibits cell wall assembly by inhibiting expression of cell wall surface layer protein based on inhibition of fungal GPI biosynthesis, while also preventing pathogens from demonstrating pathogenicity by inhibiting adhesion of fungi to cells, thereby allowing it to demonstrate efficacy against the onset, advance and persistence of infections.

Although the pharmaceutical composition of the present invention is a pharmaceutical composition comprising 3-(3-(4-(pyridin-2-yloxymethyl)-benzyl)-isoxazol-5-yl)-pyridin-2-ylamine or 2-amino-1-((phosphonooxy) methyl)-3-(3-((4-((2-pyridinyloxy)methyl)phenyl)methyl)-5-isoxazolyl)-pyridinium, or a salt thereof in combincation with an antifungal agent, the 3-(3-(4-(pyridin-2-yloxymethyl)-benzyl)-isoxazol-5-yl)-pyridin-2-ylamine or 2-amino-1-((phosphonooxy)methyl)-3-(3-((4-((2-pyridinyloxy)methyl)phenyl)methyl)-5-isoxazolyl)-pyridinium, or a salt thereof may be a compound or a single salt thereof, or a compound or mixture of salts thereof.

The term "antifungal agent" used in the present description refers to a preventive agent and/or therapeutic agent for mycosis, and is at least one compound selected from the group consisting of a triazole antifungal agent, an imidazole antifungal agent, an echinocandin antifungal agent, a polyene antifungal agent, a fluoropyridine antifungal agent, T-2307 (4-(3-(1-(3-(4-amidinophenoxy)propyl)piperidin-4-yl)propoxy)benzamidine, described in WO 2006/003881 and Antimicrob. Agents Chemother., 2008, Vol. 52, pp. 1318-1324) and FG3622 (2-(1,5-dimethyl-3-phonyl-1 H-pyrrol-2-yl)-N-(4-(4-(4,6-dimethylpyridin-2-yl)-piperazin-1-yl)phenyl)-2-oxo-acetoamide, described in WO 2009/130481), or a salt thereof.

The antifungal agent used in the pharmaceutical composition of the present invention may be at least one compound or a single salt thereof or a compound or mixtures of salts thereof selected from antifungal agents.
The antifungal agent is preferably a triazole antifungal agent, an echinocandin antifungal agent, a polyene antifungal agent or a fluoropyridine antifungal agent, and more preferably a triazole antifungal agent, an echinocandin antifungal agent or a polyene antifungal agent.

Although there are no particular limitations thereon, examples of a triazole antifungal agent include fluconazole, fosfluconazole, itraconazole, voriconazole, posaconazole, isavuconazole, ravuconazole, ((2R,3R)-3-(4-(4-cyanophenyl)thiazol-2-yl)-2-(2,4-difluorophenyl)-1-(1H-1,2,4-triazol-1-yl)butan-2-yloxy)methyl dihydrogen phosphate (described in WO 2001/052852) and albaconazole. Preferable examples of a triazole antifungal agent include fluconazole, voriconazole and ravuconazole. Preferable examples of a triazole antifungal agent for Candida in particular include fluconazole, voriconazole and ravuconazole, and these antifungal agents are more preferable for Candida tropicalis in particular.

Although there are no particular limitations thereon, examples of an imidazole antifungal agents include miconazole and ketoconazole.

Although there are no particular limitations thereon, examples of an echinocandin antifungal agents include caspofungin, micafungin, anidulafungin, aminocandin. Preferable examples of an echinocandin antifungal agent include caspofungin and micafungin. Preferable examples of an echinocandin antifungal agent for Aspergillus in particular include caspofungin and micafungin, while these antifungal agents are more preferable for Aspergillus fumigatus or Aspergillus flavus in particular.

In a mouse Aspergillus fumigatus infection model, in addition to micafungin, ravuconazole and voriconazole also demonstrate synergistic combinantion effects and are more preferable, while in a mouse Aspergillus flavus infection model, in addition to caspofungin and micafungin, amphotericin B also demonstrates synergistic combined effects and is more preferable.

Although there are no particular limitations thereon, examples of a polyene antifungal agent include amphotericin B and nystatin. A preferable example of a polyene antifungal agent is amphotericin B.

Although there are no particular limitations thereon, examples of a fluoropyridine antifungal agent include flucytosine.

In the pharmaceutical composition of the present invention, 3-(3-(4-(pyridin-2-yloxymethyl)-benzyl)-isoxazol-5-yl)-pyridin-2-ylamine or 2-amino-1-((phosphonooxy)methyl)-3-(3-((4-((2-pyridinyloxy)methyl)phenyl)methyl)-5-isoxazolyl)-pyridinium, or a salt thereof, and an antifungal agent are used as active ingredients.

In the present invention, a medicament comprising 3-(3-(4-(pyridin-2-yloxymethyl)-benzyl)-isoxazol-5-yl)-pyridin-2-ylamine or 2-amino-1-((phosphonooxy)methyl)-3-(3-((4-((2-pyridinyloxy)methyl)phenyl)methyl)-5-isoxazolyl)-pyridinium, or a salt thereof is used as a medicament for combination use with at least one compound selected from the group consisting of a triazole antifungal agent, an imidazole antifungal agent, an echinocandin antifungal agent, a polyene antifungal agent, a fluoropyridine antifungal agent, T-2307 and FG3622, or a salt thereof, while a medicament comprising an antifungal agent as an active ingredient thereof is used as a medicament for combination use with 3-(3-(4-(pyridin-2-yloxymethyl)-benzyl)-isoxazol-5-yl)-pyridin-2-ylamine or 2-amino-1-((phosphonooxy)methyl)-3-(3-((4-((2-pyridinyloxy)methyl)phenyl)methyl)-5-isoxazolyl)-pyridinium, or a salt thereof.

A formulation comprising an antifungal agent as an active ingredient thereof, such as an amphotericin B lipid formulation in the manner of an amphotericin B liposome formulation in the case of using amphotericin B for the antifungal agent, can also be used for these antifungal agents.

Although the pharmaceutical composition of the present invention is a pharmaceutical composition comprising a combination, there are no particular limitations thereon provided it is a pharmaceutical composition that allows 3-(3-(4-(pyridin-2-yloxymethyl)-benzyl)-isoxazol-5-yl)-pyridin-2-ylamine or 2-amino-1-((phosphonooxy)methyl)-3-(3-((4-((2-pyridinyloxy)methyl)phenyl)methyl)-5-isoxazolyl)-pyridinium, or a salt thereof, and an antifungal agent to be administered simultaneously or separately. The pharmaceutical composition of the present invention may be a combination medicament, and each active ingredient can be administered simultaneously or separately.
Simultaneous administration refers to combination use by administering 3-(3-(4-(pyridin-2-yloxymethyl)-benzyl)-isoxazol-5-yl)-pyridin-2-ylamine or 2-amino-1-((phosphonooxy)methyl)-3-(3-((4-((2-pyridinyloxy)methyl)phenyl)methyl)-5-isoxazolyl)-pyridinium, or a salt thereof, and an antifungal agent at the same time, and a drug in the mixed form may be administered, a mixture prepared at the time of use during administration may be administered, or drugs in different forms during administration may be administered at the same time. In the case of using by administering simultaneously, administration may be carried out using different paths or administration may be carried out using the same path, and the administered drug form may be the same or different.
In addition, when administering 3-(3-(4-(pyridin-2-yloxymethyl)-benzyl)-isoxazol-5-yl)-pyridin-2-ylamine or 2-amino-1-((phosphonooxy)methyl)-3-(3-((4-((2-pyridinyloxy)methyl)phenyl)methyl)-5-isoxazolyl)-pyridinium, or a salt thereof, and an antifungal agent at the same time in different forms during administration, different formulations may be administered all at once or may be administered successively.
Administering separately refers to combination use of 3-(3-(4-(pyridin-2-yloxymethyl)-benzyl)-isoxazol-5-yl)-pyridin-2-ylamine or 2-amino-1-((phosphonooxy)methyl)-3-(3-((4-((2-pyridinyloxy)methyl)phenyl)methyl)-5-isoxazolyl)-pyridinium, or a salt thereof, and an antifungal agent by stipulating the dosage method and dose according to the properties of each compound and administering at intervals, administration may be carried out using different paths or administration may be carried out using the same path, and the administered drug form may be the same or different. Administering at intervals may be administering before meals on the one hand or administering during or after meals on the other hand, or administering once a day on the one hand or 2 to 3 times per day on the other hand (in this case, during administration once a day on the one hand, administration may be performed simultaneously on the other hand).

As a result of the pharmaceutical composition of the present invention comprising 3-(3-(4-(pyridin-2-yloxymethyl)-benzyl)-isoxazol-5-yl)-pyridin-2-ylamine or 2-amino-1-((phosphonooxy)methyl)-3-(3-((4-((2-pyridinyloxy)methyl)phenyl)methyl)-5-isoxazolyl)-pyridinium, or a salt thereof, and an antifungal agent, it has superior antifungal activity and is extremely useful as a preventive or therapeutic agent for mycosis.
As a result of using by combining 3-(3-(4-(pyridin-2-yloxymethyl)-benzyl)-isoxazol-5-yl)-pyridin-2-ylamine or 2-amino-1-((phosphonooxy)methyl)-3-(3-((4-((2-pyridinyloxy)methyl)phenyl)methyl)-5-isoxazolyl)-pyridinium, or a salt thereof, and an antifungal agent, synergistic combination effects are obtained in an antifungal activity test, for example.

The pharmaceutical composition of the present invention demonstrates antifungal activity, and although there are no particular limitations on fungi, examples thereof include Candida such as Candida albicans, Candida glabrata, Candida parapsilosis, Candida tropicalis or Candida krusei, and Aspergillus such as Aspergillus fumigatus, Aspergillus flavus, Aspergillus terreus, Aspergillus niger or Aspergillus nidulans.

Although there are no particular limitations thereon, examples of diseases for which the pharmaceutical composition of the present invention is used include candidiasis such as systemic candidiasis, oral candidiasis, esophageal candidiasis, pulmonary candidiasis or urinary candidiasis, and aspergillosis such as pulmonary aspergillosis or central nervous system aspergillosis.

The pharmaceutical composition of the present invention may be in the form of a mixture of 3-(3-(4-(pyridin-2-yloxymethyl)-benzyl)-isoxazol-5-yl)-pyridin-2-ylamine or 2-amino-1-((phosphonooxy)methyl)-3-(3-((4-((2-pyridinyloxy)methyl)phenyl)methyl)-5-isoxazolyl)-pyridinium, or a salt thereof, and an antifungal agent, or may each be separate pharmaceutical forms. In the case of being in separate pharmaceutical forms, the pharmaceutical composition may be in the pharmaceutical form of a mixture by preparing at the time of use, or the separate pharmaceutical forms may be administered separately.

The pharmaceutical composition of the present invention may also be a kit, and there are no particular limitations thereon provided the kit is provided with 3-(3-(4-(pyridin-2-yloxymethyl)-benzyl)-isoxazol-5-yl)-pyridin-2-ylamine or 2-amino-1-((phosphonooxy)methyl)-3-(3-((4-((2-pyridinyloxy)methyl)phenyl)methyl)-5-isoxazolyl)-pyridinium, or a salt thereof, and an antifungal agent.
In the kit, 3-(3-(4-(pyridin-2-yloxymethyl)-benzyl)-isoxazol-5-yl)-pyridin-2-ylamine or 2-amino-1-((phosphonooxy)methyl)-3-(3-((4-((2-pyridinyloxy)methyl)phenyl)methyl)-5-isoxazolyl)-pyridinium, or a salt thereof, and an antifungal agent may be packaged separately or may be packaged after mixing.
In the case of using as an injection formulation, a solvent such as physiological saline may also be comprised in the kit, and the kit may comprise other ingredients according to the administration form.
In addition, the kit may also comprise a packaging container, instruction manual, package insert and the like, and a label may be indicated to the effect that the kit comprises an antifungal agent.

The pharmaceutical composition of the present invention can be formulated in the form of tablets, powder, grains, granules, coated tablets, capsules, syrup, lozenges, inhalant, suppositories, injections, ointments, ophthalmic ointments, tape, eye drops, nose drops, ear drops, poultices or lotions and the like in accordance with commonly used methods by using a mixture of the active ingredients used in the present invention as the same formulation or as different formulations. The 3-(3-(4-(pyridin-2-yloxymethyl)-benzyl)-isoxazol-5-yl)-pyridin-2-ylamine or 2-amino-1-((phosphonooxy)methyl)-3-(3-((4-((2-pyridinyloxy)methyl)phenyl)methyl)-5-isoxazolyl)-pyridinium, or a salt thereof, and an antifungal agent may be comprised separately in the same formulation or different formulations, may be mixed and used in the same formulation, or may be present separately in the same formulation.

Commonly used excipients, binders, lubricants, colorants and correctives and the like, as well as stabilizers, emulsifiers, absorption promoters, surfactants, pH adjusters, antiseptics and antioxidants used as necessary, can be used for formulation, and ingredients typically used as raw materials of pharmaceutical formulations are incorporated and formulated according to ordinary methods.

When producing an oral formulation, for example, powders, grains, granules, tablets, coated tablets or capsules and the like are formulated in accordance with ordinary methods after having adding the active ingredient compounds used in the present invention and an excipient, and further adding a binder, a disintegrating agent, a lubricant, a colorant and a corrective and the like as necessary.

Although there are no particular limitations thereon, examples of ingredients used in formulation include vegetable oils such as soybean oil, beef tallow or synthetic glycerides; hydrocarbons such as liquid paraffin, squalane or solid paraffin; ester oils such as octyldodecyl myristate or isopropyl myristate; higher alcohols such as cetostearyl alcohol or behenyl alcohol; silicon resins; silicon oils; surfactants such as polyoxyethylene fatty acid esters, sorbitan fatty acid esters, glycerin fatty acid esters, polyoxyethylene sorbitan fatty acid esters, polyoxyethylene hydrogenated castor oil or polyoxyethylene-polyoxypropylene block copolymers; water-soluble polymers such as hydroxyethyl cellulose, polyacrylic acid, carboxyvinyl polymer, polyethylene glycol, polyvinyl pyrrolidone or methyl cellulose; lower alcohols such as ethanol or isopropanol; polyvalent alcohols such as glycerin, propylene glycol, dipropylene glycol or sorbitol; sugars such as glucose or sucrose; inorganic powders such as silicic anhydride, aluminum magnesium silicate or aluminum silicate; and purified water. Examples of excipients include lactose, cornstarch, saccharose, glucose, mannitol, sorbitol, crystalline cellulose and silicon dioxide, examples of binders include polyvinyl alcohol, polyvinyl ether, methyl cellulose, ethyl cellulose, gum arabic, tragacanth, gelatin, shellac, hydroxypropyl methyl cellulose, hydroxypropyl cellulose, polyvinyl pyrrolidone, polypropylene glycol-polyoxyethylene block polymer and meglumine, examples of disintegrating agents comprise starch, agar, powdered gelatin, crystalline cellulose, calcium carbonate, sodium bicarbonate, calcium citrate, dextrin, pectin and calcium carboxymethyl cellulose, examples of lubricants include magnesium stearate, talc, polyethylene glycol, silica and hydrogenated vegetable oils, examples of colorants include colorants permitted to be added to pharmaceuticals, and examples of correctives include powdered cocoa, menthol, aromatic powders, peppermint oil, borneol and powdered cinnamon. Tablets and granules may be coated with sugar or other suitable coating as necessary.

In addition, when producing a liquid formulation such as a syrup or injection formulation, the liquid formulation is formulated in accordance with ordinary methods by adding a pH adjuster, a solvent or a tonicity agent and the like to a compound used as an active ingredient in the present invention, and adding a solubilizing agent or a stabilizer and the like as necessary.
There are no particular limitations on the method used when producing an external formulation, and an external formulation can be produced in accordance with ordinary methods. Namely, various types of raw materials ordinarily used in pharmaceuticals, quasi drugs and cosmetics and the like can be used as base raw materials used in formulation. Specific examples of base raw materials used include animal and vegetable oils, mineral oils, ester oils, waxes, higher alcohols, fatty acids, silicon oil, surfactants, phospholipids, alcohols, polyvalent alcohols, water-soluble polymers, clay minerals and purified water, and although pH adjusters, antioxidants, chelating agents, antiseptics and anti-mold agents, colorants, fragrances and the like can be further added as necessary, base raw materials for use as an external formulation are not limited thereto. In addition, ingredients having differentiation-inducing action, circulation accelerators, disinfectants, antiphlogistic agents, cell activating agents, vitamins, amino acids, moisturizers and keratolytic agents and the like can also be incorporated as necessary. The added amounts of the above-mentioned base raw materials are amounts that result in a concentration normally set in the production of external formulations.

In the case of administering a compound used as an active ingredient in the present invention, there are no particular limitations on the form thereof, and may be administered orally or parenterally by an ordinarily used method. For example, the compound can be administered by formulating in the form of a tablet, powder, granules, capsule, syrup, lozenge, inhalant, suppository, injection, ointment, ophthalmic ointment, tape, eye drops, nose drops, ear drops, poultice or lotion.

The dose of each active ingredient for combination use as the pharmaceutical composition of the present invention can be suitably selected corresponding to the degree of symptoms, age, gender, body weight, type of administration form and salt, specific disease type and the like.

Although each dose differs considerably according to the type of disease and degree of symptoms of the patient, patient age and gender, drug sensitivity and the like, in the case of an oral formulation, the normal adult dose is 1 mg to 10000 mg, and preferably 10 mg to 2000 mg, per day administered once to several times per day. In the case of an injection formulation, the normal adult dose is 0.1 to 10000 mg, and preferably 1 mg to 2000 mg, per day.

### Examples

Although the following provides a more detailed explanation of the present invention using examples thereof, these examples are intended to be exemplary, and the present invention is not limited to only these examples.

The following compounds were used as test compounds. 3-(3-(4-(pyridin-2-yloxymethyl)-benzyl)-isoxazol-5-yl)-pyridin-2-ylamine (Test Compound 1)
2-amino-1-((phosphonooxy)methyl)-3-(3-((4-((2-pyridinyloxy)methyl)phenyl) methyl)-5-isoxazolyl)-pyridinium (Test Compound 2)
Test Compound 1 was produced in accordance with the method described in WO 2007/052615. In addition, Test Compound 2 was produced in accordance with the method described in WO 2009/084621.

Ravuconazole produced in accordance with the method described in Patent Publication JP-A-H8-20578 was used as an antifungal agent. In other antifungal agents for in vitro testing, extracts of fluconazole (trade name: Diflucan, Pfizer Inc.), voriconazole (trade name: Vfend, Pfizer Inc.) and micafungin (trade name: Funguard, Astellas Pharma Inc.) were used and caspofungin (trade name: Cancidas, Merck & Co.) and amphotericin B (Sigma-Aldrich Corp.) were used. Antifungal agents for in vivo testing, voriconazole (trade name: Vfend, Pfizer Inc.) was used as an extract and micafungin (trade name: Funguard, Astellas Pharma Inc.), caspofungin (trade name: Cancidas, Merck & Co.), amphotericin B (trade name: Fungizone, Bristol-Myers Co.) and liposomal amphotericin B (trade name: Ambisome, Dainippon Sumitomo Pharma Co., Ltd.) were used. Values were indicated as converted values in cases in which commercially available formulations were used directly for in vitro and in vivo testing.

A clinically isolated strain retained by the Medical Mycology Research Center of Chiba University (Code No. IFM) and a clinically isolated strain retained by the Life Science Research Center of Gifu University (Code No. E) were used as Candida and Aspergillus strains.

### I. Measurement of In Vitro Combination Effects for Anti-Candida Activity and Anti-Aspergillus Activity

### <Method>

### (1) Preparation of Fungus Liquids

### Candida Strain

Fungus liquid obtained by static culturing at 35°C for 48 hours in Sabouraud Dextrose liquid medium (SDB, Becton, Dickinson and Co.) was diluted with RPMI 1640 medium (Nissui Pharmaceutical Co., Ltd.) containing 0.165 M 3-(N-morpholino)propanesulfonic acid (MOPS) to prepare a fungus liquid having a fungus concentration of 1 × 10³ cells/mL to 2 × 10³ cells/mL.

### Aspergillus Strain

An Aspergillus strain stored frozen at -80°C, or spores obtained by static culturing at 35°C for 1 week in Potato Dextrose agar medium (PDA, Eiken Chemical Co., Ltd.) followed by scraping from the container with a metal loop, was diluted with RPMI 1640 medium containing 0.165 M MOPS to prepare a fungus liquid having a fungus concentration of 0.75 × 10⁴ cells/mL to 1.33 × 10⁴ cells/mL.

### (2) Preparation of Drug Dilution Plates

Test Compound 1 (0.0005 µg/mL to 1.56 µg/mL) in the horizontal rows (12 wells) of a 96-well flat-bottom plate, and an antifungal agent in the form of ravuconazole (0.0015 µg/mL to 6.25 µg/mL), fluconazole (0.008 µg/mL to 32 µg/mL), voriconazole (0.0002 µg/mL to 8 µg/mL), micafungin (0.0005 µg/mL to 0.25 µg/mL), caspofungin (0.0005 µg/mL to 1 µg/mL) or amphotericin B (0.008 µg/mL to 8 µg/mL) in the vertical rows (8 wells) of the flat-bottom plate were prepared by diluting so as to blend together using RPMI 1640 medium containing 0.165 M MOPS to obtain a control group, a single Test Compound 1 group, each one of antifungal agents groups and combination groups of Test Compound 1 + each one of antifungal agents.

### (3) Inoculation and Culturing of Fungus Liquid

The fungus liquids prepared in (1) were inoculated into the wells of 96-well flat-bottom plate containing 20 µL/well to 75 µL/well of the diluted test drug liquids prepared in (2) at 80 µL/well to 125 µL/well, followed by aerobically static culturing for 25 hours to 48 hours at 35°C.

### (4) Measurement of MIC

The minimum concentration at which fungal growth appeared at a glance to be clearly inhibited in comparison with the control, or the minimum concentration at which turbidity at an absorbance of 660 nm decreased to 50% or less of the control, was taken to be the minimum inhibitory concentration (MIC).

### (5) Calculation of Fractional Inhibitory Concentration (FIC) Index

Combination effects of Test Compound 1 and the antifungal agents were measured with the checkerboard method by micro dilution.
Interaction between both drugs was assessed by calculating the FIC index using the calculation formula indicated below.
The FIC index was calculated for each combination of antifungal agent concentrations that inhibited fungal growth, and the minimum value of the resulting FIC index was used as a combination FIC index.
A combination FIC index of 0.5 or less was evaluated as indicating synergistic action, that of 0.5 to 1 or less was evaluated as indicating additive action, and that of 1 to 4 or less was evaluated as indicating indifferent action. In addition, in the case an FIC index of greater than 4 was observed for a certain combination of antifungal agent combinations, this was evaluated as indicating antagonistic action regardless of FIC index values for other concentration combinations.

### (Calculation Formula)

FIC Index = (MIC value of drug A during combination use/MIC value of drug A when used alone) + (MIC value of drug B during combination use/MIC value of drug B when used alone)

As a result, as shown in Tables 1 and 2, Test Compound 1 demonstrated synergistic or additive effects with each type of antifungal agent for in vitro antifungal activity, and since the combinations clearly did not demonstrate antagonistic action, they were recognized to be combinations that allow combination use.
Test Compound 1 demonstrated definitive synergistic effects with azole drugs against Candida and with echinocandin drugs against Aspergillus in particular.

**Table 1 In Vitro Combination Effects with Various Antifungal Agents Against Candida and Aspergillus**

| Fungus Species (No of strains) | Compound for combination use | Ratio indicating following effects of combination use with Test Compound 1 (%) | | | |
|---|---|---|---|---|---|
| | | Synergistic | Additive | indifferent | Antagonistic |
| C. albicans | Fluconazole | 10 | 80 | 10 | 0 |
| (20) | Voriconazole | 15 | 75 | 10 | 0 |
| | Micafungin | 0 | 80 | 20 | 0 |
| | Caspofungin | 0 | 85 | 15 | 0 |
| | Amphotericin B | 5 | 85 | 10 | 0 |
| C. tropicalis (10) | Fluconazole | 90 | 10 | 0 | 0 |
| | Voriconazole | 100 | 0 | 0 | 0 |
| | Micafungin | 0 | 100 | 0 | 0 |
| | Caspofungin | 0 | 90 | 10 | 0 |
| | Amphotericin B | 0 | 90 | 10 | 0 |
| C. glabrata (10) | Fluconazole | 0 | 90 | 10 | 0 |
| | Voriconazole | 30 | 60 | 10 | 0 |
| | Micafungin | 0 | 70 | 30 | 0 |
| | Caspofungin | 0 | 100 | 0 | 0 |
| | Amphotericin B | 30 | 70 | 0 | 0 |
| A. fumigatus (18) | Voriconazole | 6 | 28 | 67 | 0 |
| | Micafungin | 100 | 0 | 0 | 0 |
| | Caspofungin | 78 | 22 | 0 | 0 |
| | Amphotericin B | 0 | 33 | 67 | 0 |
| A. flavus (4) | Voriconazole | 0 | 50 | 50 | 0 |
| | Micafungin | 100 | 0 | 0 | 0 |
| | Caspofungin | 75 | 25 | 0 | 0 |
| | Amphotericin B | 0 | 25 | 75 | 0 |

In Table 1, combination use of Test Compound 1 and fluconazole or voriconazole demonstrated synergistic effects against Candida albicans IFM49738, which is resistant to voriconazole and fluconazole, or in other words, is a strain that is resistant to azole antifungal agents. Combination use of Test Compound 1 with fluconazole or voriconazole demonstrated synergistic effects against each of the resistant strains consisting of Candida tropicalis strain E83037, E83039 and E83043, which are resistant to voriconazole and fluconazole, or in other words, are strains that are resistant to azole antifungal agents, Candida tropicalis strain E83042, which is resistant to fluconazole, and Candida tropicalis strain E83046, which is resistant to voriconazole. In addition, combination use of Test Compound 1 with amphotericin B demonstrated synergistic effects against Candida glabrata strain IFM46888, which is resistant to fluconazole.

**Table 2 In Vitro Combination Effects with Ravuconazole Against Candida and Aspergillus**

| Fungus Species (No of strains) | Compound for combination use | Ratio indicating following effects of combination use with Test Compound 1 (%) | | | |
|---|---|---|---|---|---|
| | | Synergistic | Additive | Indifferent | Antagonistic |
| C. albicans (9) | Ravuconazole | 44 | 56 | 0 | 0 |
| C. tropicalis (5) | Ravuconazole | 100 | 0 | 0 | 0 |
| C. glabrata (5) | Ravuconazole | 40 | 60 | 0 | 0 |
| A. fumigatus (16) | Ravuconazole | 0 | 6 | 94 | 0 |
| A. flavus (4) | Ravuconazole | 0 | 25 | 75 | 0 |

In Table 2, combination use of Test Compound 1 with ravuconazole demonstrated synergistic effects against Candida albicans strain IFM49738, which is resistant to ravuconazole and the like, or in other words, is a strain that is resistant to azole antifungal agents. Combination use of Test Compound 1 with ravuconazole demonstrated synergistic effects against Candida tropicalis strains E83037, E83038, E83039, E83040 and E83041, which are resistant to ravuconazole and the like, or in other words, are strains that are resistant to azole antifungal agents.

### II. Measurement of In Vivo Combination Effects in Aspergillus Respiratory Tract Infection Experiment System

### <Method>

### (1) Compromising of Mice

Female DBA/2N mice (age 8 weeks, Charles River Laboratories Japan, Inc.) were subcutaneously administered 200 mg/kg of 5-fluorouracil 5 or 6 days prior to infection in order to put the mice into a compromised state.

### (2) Test Aspergillus Strains

A. fumigatus strain IFM52108 and A. flavus strain IFM50915 were used.

### (3) Preparation of Inoculation Fungus Liquids

A liquid containing spores of A. fumigatus strain IFM52108 stored at -80°C was applied to Potato Dextrose agar medium (PDA, Eiken Chemical Co., Ltd.) followed by static culturing for 5 to 7 days at 35°C and suspending in sterile physiological saline containing 0.05% Tween 80.
The number of spores was counted using a hemocytometer, and the liquid was diluted with sterile physiological saline containing 0.05% Tween 80 to a concentration of 1.2 × 10⁶ cells/mL to 2.5 × 10⁶ cells/mL to obtain an inoculation fungus liquid.
A liquid containing spores of A. flavus strain IFM50915 stored at -80°C was thawed, and the number of spores was counted using a hemocytometer followed by diluting with sterile physiological saline containing 0.05% Tween 80 to a concentration of 0.6 × 10⁶ cells/mL to 1.0 × 10⁶ cells/mL to obtain an inoculation fungus liquid.

### (4) Infection

50 µl of inoculation fungus liquid were inoculated into the nasal cavities of 9-week-old mice at 0.3 × 10⁵ cells/mouse to 1.25 × 10⁵ cells/mouse under Ketalar anesthesia.

### (5) Treatment

0.1 mL to 0.2 mL of each drug solution (dissolved in sterile physiological saline or 5% glucose solution, or dissolved in sterile physiological saline or 5% glucose solution containing 6.5% dimethylsulfoxide and 3.5% Tween 80) was administered to the individual and combination dose groups either into the peritoneal cavity or into the stomach using a gastric cannula corresponding to the administration route of the drug for 3 to 4 days at the rate of 1 to 2 times per day starting 0.5 hours to 1 hour after inoculation of fungus.
There were 5 to 9 animals in each group.

### (6) Assessment of Results

Infection protection results were assessed on survival rate 14 days after infection.
As a result, as shown in Tables 3 to 5, Test Compound 1 and Test Compound 2 clearly demonstrated combination effects with various types of azole, echinocandin and polyene antifungal agents in an Aspergillus respiratory tract infection model.

**Table 3 Combination Effects of Test Compound 1 in A. Fumigatus Respiratory Tract Infection Model**

| Administered Compound | Test Compound 1 | | RVCZ | | Test Compound 1 10 mg/kg + RVCZ 5 mg/kg | Test Compound 1 10 mg/kg + RVCZ 10 mg/kg |
|---|---|---|---|---|---|---|
| | 10 mg/kg | 20 mg/kg | 5 mg/kg | 10 mg/kg | | |
| 14-davy survival rate (%) | 0 | 20 | 0 | 40 | 60 | 80 |

| Administered Compound | Test Compound 1 | | MCFG | | Test Compound 1 10 mg/kg + MCFG 1 mg/kg | Test Compound 1 10 mg/kg + MCFG 2 mg/kg |
|---|---|---|---|---|---|---|
| | 10 mg/kg | 20 mg/kg | 1 mg/kg | 2 mg/kg | | |
| 14-day survival rate (%) | 0 | 20 | 0 | 40 | 60 | 100 |

| Administered Compound | Test Compound 1 | | VRCZ | | Test Compound 1 10 mg/kg + VRCZ 2 mg/kg | Test Compound 1 20 mg/kg + VRCZ 2 mg/kg |
|---|---|---|---|---|---|---|
| | 10 mg/kg | 20 mg/kg | 1 mg/kg | 2 mg/kg | | |
| 14-day survival rate (%) | 40 | 40 | 0 | 20 | 60 | 80 |

**Table 4 Combination Effects of Test Compound 1 in A. Flavus Respiratory Tract Infection Model**

| Administered Compound | Test Compound 1 | | RVCZ | | Test Compound 1 10 mg/kg + RVCZ 2.5 mg/kg | Test Compound 1 10 mg/kg + RVCZ 5 mg/kg |
|---|---|---|---|---|---|---|
| | 5 mg/kg | 10 mg/kg | 2.5 mg/kg | 5 mg/kg | | |
| 14-day survival rate (%) | 20 | 60 | 40 | 80 | 100 | 100 |

| Administered Compound | Test Compound 1 | | MCFG | | Test Compound 1 5 mg/kg + MCFG 0.25 mg/kg | |
|---|---|---|---|---|---|---|
| | 5 mg/kg | | 0.5 mg/kg | | | |
| 14-day survival rate (%) | 12.5 | | 50 | | 100 | |

| Administered Compound | Test Compound 1 | | CSFG | | Test Compound 1 8 mg/kg + CSFG 0.5 mg/kg | Test Compound 1 16 mg/kg + CSFG 0.25 mg/kg |
|---|---|---|---|---|---|---|
| | 8 mg/kg | 16 mg/kg | 0.25 mg/kg | 0.5 mg/kg | | |
| 14-day survival rate (%) | 0 | 80 | 20 | 60 | 100 | 100 |

| Administered Compound | Test Compound 1 | | AMPH | | Test Compound 1 10 mg/kg + AMPH 0.5 mg/kg | Test Compound 1 10 mg/kg + AMPH 1 mg/kg |
|---|---|---|---|---|---|---|
| | 5 mg/kg | 10 mg/kg | 0.5 mg/kg | 1 mg/kg | | |
| 14-day survival rate (%) | 20 | 60 | 20 | 0 | 100 | 100 |

**Table 5 Combination Effects of Test Compound 2 in A. Flavus Respiratory Tract Infection Model**

| Administered Compound | Test Compound 2 | | CSFG | | Test Compound 2 2.5 mg/kg + CSFG 0.1 mg/kg | Test Compound 2 5 mg/kg + CSFG 0.2 mg/kg |
|---|---|---|---|---|---|---|
| | 2.5 mg/kg | 5 mg/kg | 0.1 mg/kg | 0.2 mg/kg | | |
| 14-day survival rate (%) | 0 | 50 | 37.5 | 50 | 87.5 | 100 |

| Administered Compound | Test Compound 2 | | L-AmB | | Test Compound 2 5 mg/kg + L-AmB 5 mg/kg | |
|---|---|---|---|---|---|---|
| | 5 mg/kg | | 5 mg/kg | | | |
| 14-day survival rate (%) | 0 | | 0 | | 60 | |

| Administered Compound | Test Compound 2 | | MCFG | | Test Compound 2 5 mg/kg + MCFG 0.25 mg/kg | Test Compound 2 10 mg/kg + MCFG 0.5 mg/kg |
|---|---|---|---|---|---|---|
| | 5 mg/kg | 10 mg/kg | 0.25 mg/kg | 0.5 mg/kg | | |
| 14-day survival rate (%) | 22.2 | 55.6 | 0 | 33.3 | 66.7 | 100 |

In Tables 3 to 5, RVCZ represents ravuconazole, MCFG represents micafungin, VRCZ represents voriconazole, CSFG represents caspofungin, AMPH represents amphotericin B and L-AmB represents liposomal amphotericin B.

### Industrial Applicability

The pharmaceutical composition of the present invention has superior antifungal activity, and has industrial applicability as a preventive or therapeutic agent for mycosis.

## Claims

1. A pharmaceutical composition comprising 3-(3-(4-(pyridin-2-yloxymethyl)-benzyl)-isoxazol-5-yl)-pyridin-2-ylamine or 2-amino-1-((phosphonooxy)methyl)-3-(3-((4-((2-pyridinyloxy)methyl)phenyl)methyl)-5-isoxazolyl)-pyridinium, or a salt thereof in combination with an antifungal agent, wherein the antifungal agent is at least one compound selected from the group consisting of a triazole antifungal agent, an imidazole antifungal agent, an echinocandin antifungal agent, a polyene antifungal agent, a fluoropyridine antifungal agent, T-2307 and FG3622, or a salt thereof.

2. The pharmaceutical composition according to claim 1, wherein the antifungal agent is at least one compound selected from a triazole antifungal agent, an echinocandin antifungal agent, a polyene antifungal agent and a fluoropyridine antifungal agent, or a salt thereof.

3. The pharmaceutical composition according to claim 1 or 2, wherein the antifungal agent is at least one compound selected from a triazole antifungal agent, an echinocandin antifungal agent and a polyene antifungal agent, or a salt thereof.

4. The pharmaceutical composition according to claim 1, wherein the antifungal agent is at least one compound selected from the group consisting of fluconazole, fosfluconazole, itraconazole, voriconazole, posaconazole, isavuconazole, ravuconazole, ((2R,3R)-3-(4-(4-cyanophenyl)thiazol-2-yl)-2-(2,4-difluorophenyl)-1-(1H-1,2,4-triazol-1-yl)butan-2-yloxy)methyl dihydrogen phosphate, albaconazole, miconazole, ketoconazole, caspofungin, micafungin, anidulafungin, aminocandin, amphotericin B, nystatin and flucytosine, or a salt thereof.

5. The pharmaceutical composition according to any one of claims 1 to 4, wherein the antifungal agent is at least one compound selected from the group consisting of fluconazole, voriconazole, ravuconazole, caspofungin, micafungin and amphotericin B, or a salt thereof.

6. The pharmaceutical composition according to any one of claims 1 to 5, which is for use for prevention and/or treatment of mycosis.

7. The pharmaceutical composition according to any one of claims 1 to 6, wherein a fungus is Candida or Aspergillus.

8. The pharmaceutical composition according to claim 6 or 7, wherein the mycosis is systemic candidiasis, oral candidiasis, esophageal candidiasis, pulmonary candidiasis, urinary candidiasis, pulmonary aspergillosis or central nervous system aspergillosis.

9. The pharmaceutical composition according to any one of claims 1 to 8, which is a kit.

10. A use of 3-(3-(4-(pyridin-2-yloxymethyl)-benzyl)-isoxazol-5-yl)-pyridin-2-ylamine or 2-amino-1-((phosphonooxy)methyl)-3-(3-((4-((2-pyridinyloxy)methyl)phenyl)methyl)-5-isoxazolyl)-pyridinium, or a salt thereof, and an antifungal agent for the preparation of a pharmaceutical composition, wherein the antifungal agent is at least one compound selected from the group consisting of a triazole antifungal agent, an imidazole antifungal agent, an echinocandin antifungal agent, a polyene antifungal agent, a fluoropyridine antifungal agent, T-2307 and FG3622, or a salt thereof.

11. A use of 3-(3-(4-(pyridin-2-yloxymethyl)-benzyl)-isoxazol-5-yl)-pyridin-2-ylamine or 2-amino-1-((phosphonooxy)methyl)-3-(3-((4-((2-pyridinyloxy)methyl)phenyl)methyl)-5-isoxazolyl)-pyridinium, or a salt thereof for the preparation a pharmaceutical composition in combination with an antifungal agent, wherein the antifungal agent is at least one compound selected from the group consisting of a triazole antifungal agent, an imidazole antifungal agent, an echinocandin antifungal agent, a polyene antifungal agent, a fluoropyridine antifungal agent, T-2307 and FG3622, or a salt thereof.

12. A medicament comprising 3-(3-(4-(pyridin-2-yloxymethyl)-benzyl)-isoxazol-5-yl)-pyridin-2-ylamine or 2-amino-1-((phosphonooxy)methyl)-3-(3-((4-((2-pyridinyloxy)methyl)phenyl)methyl)-5-isoxazolyl)-pyridinium, or a salt thereof for combination use with at least one compound selected from the group consisting of a triazole antifungal agent, an imidazole antifungal agent, an echinocandin antifungal agent, a polyene antifungal agent, a fluoropyridine antifungal agent, T-2307 and FG3622, or a salt thereof.

13. A medicament comprising at least one compound selected from the group consisting of a triazole antifungal agent, an imidazole antifungal agent, an echinocandin antifungal agent, a polyene antifungal agent, a fluoropyridine antifungal agent, T-2307 and FG3622, or a salt thereof for combination use with 3-(3-(4-(pyridin-2-yloxymethyl)-benzyl)-isoxazol-5-yl)-pyridin-2-ylamine or 2-amino-1-((phosphonooxy)methyl)-3-(3-((4-((2-pyridinyloxy)methyl)phenyl)methyl)-5-isoxazolyl)-pyridinium, or a salt thereof.
